# FASCICULE DE BREVET EUROPEEN

(11) **EP 2 577 283 B1**
(45) Date de publication et mention de la délivrance du brevet: **28.03.2018**
(21) Numéro de dépôt: 11723437.7
(22) Date de dépôt: 31.05.2011
(51) Int. Cl.: G01N 27/90, G01N 29/22

(54) **VÊTEMENT INTÉGRANT UN SYSTÈME DE CONTRÔLE NON DESTRUCTIF**
KLEIDUNSSTÜCK MIT INTEGRIERTEM ZERSTÖRUNGSFREIEM TESTSYSTEM
GARMENT INCLUDING A NON-DESTRUCTIVE TESTING SYSTEM

(30) Priorité: 01.06.2010 FR 1054265
(43) Date de publication de la demande: 10.04.2013
(73) Titulaire: Airbus Group SAS, 31700 Blagnac (FR)
(72) Inventeur: PAGES, Marion, F-31300 Toulouse (FR)
(86) Numéro de dépôt international: PCT/EP2011/058962
(87) Numéro de publication internationale: WO 2011/151332

(56) Documents cités:
- EP-A1- 1 213 560
- THOMAS MESTL ET AL: "MOBILE WORKER: ICT SOLUTIONS FOR THE SURVEYEOR", DET NORSKE VERITAS, XX, XX, no. 99-2043, 11 janvier 2000 (2000-01-11), pages 1-82, XP002181238,
- DUDZIAK M ET AL: "Nondestructive evaluation for crack, corrosion, and stress detection for metal assemblies and structures", PROCEEDINGS OF THE INTERNATIONAL SOCIETY FOR OPTICAL ENGINEERING (SPIE), SPIE, USA, vol. 3586, 1 janvier 1999 (1999-01-01), pages 20-31, XP009142690, ISSN: 0277-786X
- S. J. Schwartz, A. Pentland: "The smart vest: towards a next generation wearable computing platform", , vol. technical report nr. 504 juillet 1999 (1999-07), pages 1-7, XP002614614, Extrait de l'Internet: URL:http://pubs.media.mit.edu/pubs/papers/ TR-504.pdf [extrait le 2010-12-17]
- PAUL LUKOWICZ, URS ANLIKER, GERHARD TRÖSTER, STEVEN J. SCHWARTZ, RICHARD W. DEVAUL: "The WearARM Modular, Low-Power Computing Core", IEEE MICRO, vol. 21, no. 23, mai 2001 (2001-05), - juin 2001 (2001-06), pages 16-28, XP002614615, DOI: 10.1109/40.928762
- SUNKPHO J ET AL: "MIA: a wearable computer for bridge inspectors", WEARABLE COMPUTERS, 1998. DIGEST OF PAPERS. SECOND INTERNATIONAL SYMPO SIUM ON PITTSBURGH, PA, USA 19-20 OCT. 1998, LOS ALAMITOS, CA, USA,IEEE COMPUT. SOC, US, 19 octobre 1998 (1998-10-19), pages 160-161, XP010312829, DOI: DOI:10.1109/ISWC.1998.729545 ISBN: 978-0-8186-9074-7

## Description

La présente invention s'inscrit dans le domaine du contrôle non destructif, et de façon plus générale de la maintenance. Elle concerne plus particulièrement un vêtement intégrant toutes les fonctions nécessaires au contrôle non destructif.

Le vêtement selon l'invention trouve application dans tous les domaines dans lesquels il est nécessaire d'effectuer, sur des pièces ou tous types de matériaux, des contrôles par des technologies non destructives, par exemple des mesures d'épaisseur, des tests de détection de corrosion, etc. On peut citer notamment les domaines de l'aéronautique et du spatial, du bâtiment et des travaux publics, du nucléaire, du naval, cette liste n'étant nullement exhaustive.

Dans de tels domaines, pour réaliser le contrôle non destructif, les opérateurs doivent fréquemment se déplacer sur de longues distances et/ou dans des zones difficiles d'accès. Dans le domaine aéronautique notamment, les opérations d'inspection sur les lignes d'assemblage final, dont les dimensions sont de plus en plus importantes, nécessitent des déplacements sur de longues distances. Certaines opérations d'inspection dans des zones d'accès difficile telles que l'empennage d'un aéronef nécessitent l'utilisation de harnais pour amener l'opérateur dans la zone où le contrôle doit être effectué. Il en est de même par exemple pour l'inspection des lanceurs dans le domaine spatial, ou encore des ponts dans le milieu du bâtiment et des travaux publics. Dans d'autres cas, l'inspection doit être réalisée dans des zones difficilement accessibles car très confinées et étroites, par exemple dans des trous d'homme.

Les systèmes de contrôle non destructif existants se composent typiquement d'une pluralité d'éléments, principalement d'un module électronique de mesure se reliant à un ou plusieurs capteurs de mesure, qui permet de réaliser des mesures non destructives de type prédéterminé en fonction de l'application visée, par exemple par ultrasons ou par courant de Foucault ; d'une carte électronique, ou carte mère, pour la génération de signaux de commande en direction du module de mesure, et la réception et le traitement de signaux transférés depuis ce dernier ; d'une source d'alimentation électrique et d'un écran d'affichage et de commande, ces différents éléments étant reliés opérationnellement les uns aux autres par des câbles de liaison électrique et électronique. Ce matériel est encombrant, lourd (de l'ordre de 6 à 10 kg) et peu adapté à une utilisation itinérante.

A l'heure actuelle, l'ensemble de ce matériel est transporté par l'opérateur dans des valises ou à la main. Pour l'inspection proprement dite, il est fréquent que l'opérateur se voie contraint de porter l'appareillage d'un bras, l'autre bras étant utilisé pour tenir le capteur de mesure et pour effectuer le réglage des paramètres. On comprend aisément que dans certaines conditions d'environnement, la tâche de l'opérateur est alors difficile et inconfortable. Dans des cas extrêmes, ceci nuit à la qualité même de l'inspection.

La publication de Dudziak et al., dans Proc. Int. Soc. For Optical Eng., vol. 3586, 1999, pp 20-31 et le document de brevet EP 1 213 560 décrivent des systèmes de contrôle non destructif aptes à être portés sur le corps et comportant, en tant qu'éléments constitutifs, un dispositif électronique de mesure non destructive apte à être connecté à un capteur de mesure non destructive, et relié à une carte électronique de commande elle-même reliée à une source d'alimentation électrique et à un écran de visualisation et de commande, et des câbles de liaison électrique et électronique de ces éléments constitutifs les uns aux autres.

La présente invention vise à améliorer les conditions de travail des opérateurs de contrôle non destructif, et par là-même tant la qualité des inspections que la sécurité des opérateurs, et par voie de conséquence, de façon générale, la sécurité dans le domaine concerné, par exemple le domaine aéronautique.

Le contrôle non destructif est défini dans la présente description de manière classique, c'est-à-dire comme un contrôle réalisé sur des pièces ou des structures externes à l'opérateur, et visant à fournir des informations sur la santé de ces pièces ou structures, plus particulièrement sur leur intégrité et/ou leur conformité à des exigences de qualité matière, ceci sans qu'il en résulte des altérations préjudiciables à leur utilisation ultérieure. Le contrôle non destructif permet ainsi de mettre en évidence des défectuosités susceptibles d'altérer la disponibilité, la sécurité d'emploi et, plus généralement, la conformité de la pièce ou de la structure à l'usage auquel elle est destinée. A titre d'exemple, le système de contrôle non destructif selon l'invention permet ainsi de repérer des défauts non visibles sur les pièces, tels que des fissures, des délaminations dans les matériaux composites, des défauts de collage entre les pièces, ou encore un défaut de porosité d'une résine dans un matériau composite.

Le contrôle non destructif au sens de la présente invention met en oeuvre un capteur de mesure non destructive manuel, c'est-à-dire à tenir à la main pour réaliser les mesures.

A cet effet, il est proposé selon l'invention un système pour le contrôle non destructif d'une pièce selon la revendication 1.

Les différents éléments constitutifs du système selon l'invention, en particulier le dispositif électronique de mesure, sont spécifiques du contrôle non destructif et connus de l'homme du métier. Le dispositif électronique de mesure est ainsi du type apte à envoyer au capteur de mesure un signal d'excitation de sorte à générer un stimulus dans la pièce, et à traiter et interpréter un signal résultant, reçu en retour de la pièce par le capteur de mesure. Le dispositif électronique de mesure, ainsi que le capteur de mesure auquel il est destiné à être relié, sont conçus, de manière classique en elle-même, pour permettre des mesures actives de la santé de la pièce, c'est-à-dire par stimulation de cette dernière et mesure de sa réponse à cette stimulation, par opposition à une simple mesure passive d'une caractéristique physique d'un matériau (telle que sa température, son épaisseur, etc.). Le dispositif électronique de mesure comporte en particulier une partie émettrice et une partie réceptrice, conçues en fonction du type particulier de mesure non destructrice visé.

L'opérateur vêtu d'un tel vêtement n'a avantageusement plus besoin, pour se déplacer et effectuer les opérations d'inspection, de porter les éléments constitutifs du système de contrôle à bout de bras. Ses deux mains restent libres en permanence pour d'une part effectuer le paramétrage des mesures et d'autre part tenir le capteur lors de la mise en oeuvre des opérations d'inspection proprement dites, en même temps que pour assurer sa sécurité. Sa sécurité et son confort sont ainsi améliorés, et ce quel que soit l'environnement extérieur, et par voie de conséquence il en est de même pour la qualité des mesures d'inspection.

Selon une caractéristique avantageuse de l'invention, pour chaque capteur de mesure, un câble de liaison du dispositif électronique de mesure et du capteur comporte, à une extrémité de connexion au capteur, un connecteur disposé à l'extérieur de la veste, au niveau d'une manche de cette dernière, et de préférence à proximité du poignet. Une telle caractéristique s'avère particulièrement avantageuse, notamment en ce qu'elle permet à l'opérateur, au moment d'effectuer les opérations d'inspection, de connecter le capteur de mesure, qui destiné à être tenu dans la main pour la réalisation des mesures, au plus près de cette dernière.

Suivant des modes de réalisation préférés, l'invention répond en outre aux caractéristiques suivantes, mises en oeuvre séparément ou en chacune de leurs combinaisons techniquement opérantes.

Dans des modes de réalisation préférés de l'invention, le dispositif électronique de mesure, la carte électronique et la source d'alimentation électrique, ainsi que les câbles de liaison, sont disposés sur une face dite interne de la veste, qui est définie comme la face destinée à être positionnée en regard du corps d'un utilisateur, si bien que ces éléments sont avantageusement protégés de l'environnement extérieur.

L'invention prévoit en outre avantageusement que ces éléments constitutifs soient distribués sur la veste de telle sorte à répartir de manière homogène le poids à porter par l'utilisateur.

A cet égard, selon une caractéristique avantageuse de l'invention, le système comporte un dispositif de réglage de la position de la source d'alimentation électrique dans la veste. Ce dispositif permet avantageusement à l'opérateur d'ajuster la position de la source d'alimentation, qui constitue l'élément le plus lourd du système, sur la veste en fonction de sa morphologie particulière, ainsi que de la configuration de la zone à inspecter, de sorte à lui procurer le meilleur confort d'utilisation. En particulier, ce dispositif permet avantageusement à l'opérateur d'ajuster la position de la source d'alimentation électrique en cours d'inspection, par exemple pour la faire passer du dos vers le ventre, de sorte à se trouver dans la position la plus confortable possible pour réaliser l'inspection.

Préférentiellement, ce dispositif de réglage comporte des moyens de guidage du déplacement de la source d'alimentation électrique entre des positions déterminées sur la veste, et des moyens pour son immobilisation réversible dans chacune de ces positions déterminées.

De manière générale, les éléments constitutifs du système de contrôle non destructif selon l'invention sont choisis de sorte à présenter un poids, une taille et une consommation électrique les plus réduits possible.

Dans des modes de réalisation préférés de l'invention, la veste comporte une enveloppe externe et une doublure interne amovible, et le dispositif électronique de mesure, la carte électronique, la source d'alimentation électrique et les câbles de liaison sont disposés entre cette enveloppe externe et la doublure interne.

Afin de répondre à un objectif de l'invention qui est de proposer un système de contrôle non destructif présentant une ergonomie d'utilisation élevée, selon une caractéristique avantageuse, la veste comporte, sur une face dite externe opposée à une face interne destinée à être disposée en regard du corps de l'utilisateur, une poche de réception d'un écran de visualisation et de commande. La veste est en outre percée, à l'intérieur de cette poche, d'un orifice pour le passage d'un câble de liaison de l'écran et de la carte électronique. L'écran, maintenu rangé dans cette poche lors des déplacements, est alors facilement et directement accessible à l'opérateur portant la veste lorsqu'il est nécessaire d'effectuer des mesures d'inspection. Il peut alors être ôté de la poche pour être utilisé. Préférentiellement, la poche est pourvue de moyens de protection de l'écran en son intérieur. Elle est par exemple rembourrée de mousse.

Dans des modes de réalisation préférés de l'invention, en variante ou en association avec la caractéristique ci-avant, un écran de visualisation et de commande est intégré dans une manche de la veste, de sorte à être visible et utilisable à tout moment par l'opérateur portant la veste.

Selon une caractéristique avantageuse de l'invention, la veste comporte, sur une face dite externe, une pochette de réception d'un capteur de mesure, dans laquelle ce dernier peut être maintenu rangé en dehors des temps de mise en oeuvre des opérations d'inspection.

Dans des modes de réalisation préférés de l'invention, le système comporte une pluralité de capteurs, et la veste comporte une pluralité de pochettes de réception correspondantes.

Dans des modes de réalisation préférés de l'invention, le système comporte en outre des moyens d'éclairage disposés sur une face dite externe de la veste, de préférence au niveau d'une manche de cette dernière, de sorte à procurer un éclairage localisé et ciblé sur le site de l'inspection. Les moyens d'éclairage peuvent également par exemple être situés au niveau d'un col de la veste, pour obtenir une luminosité plus globale pour l'utilisateur vêtu de la veste. Une telle caractéristique s'avère notamment tout à fait avantageuse pour une inspection dans des zones confinées et sombres. Ces moyens d'éclairage sont préférentiellement contrôlés par des moyens d'allumage et d'extinction manuels disposés également sur une face externe de la veste, et connectés pour leur alimentation à la carte électronique du système.

Le vêtement intégrant un système de contrôle non destructif selon l'invention, répondant à l'une ou plusieurs des caractéristiques ci-avant, s'avère tout à fait adapté à un usage itinérant. Il offre notamment l'avantage d'une ergonomie d'utilisation élevée, tant pour les déplacements jusqu'aux sites d'inspection que pour la réalisation des mesures d'inspection elles-mêmes. Il permet de réduire le temps nécessaire à chaque contrôle, et assure notamment une augmentation du degré de sécurité pour les opérateurs et de la fiabilité des mesures effectuées

L'invention sera maintenant plus précisément décrite dans le cadre de modes de réalisation préférés, qui n'en sont nullement limitatifs, représentés sur les figures 1 à 3, dans lesquelles :
- la figure 1 représente de façon schématique la face interne d'un vêtement intégrant un système de contrôle non destructif selon un exemple de réalisation de l'invention, en position dépliée, sans câbles de liaison entre les différents éléments constitutifs ;
- la figure 2 illustre la liaison par câbles entre les différents éléments constitutifs du vêtement de la figure 1 ;
- et la figure 3 montre une vue générale externe d'un vêtement selon un exemple de réalisation de l'invention.

Le système de contrôle non destructif selon l'invention comporte un vêtement de type veste 1 dans lequel sont intégrés les différents éléments constitutifs du système permettant de réaliser des mesures d'inspection.

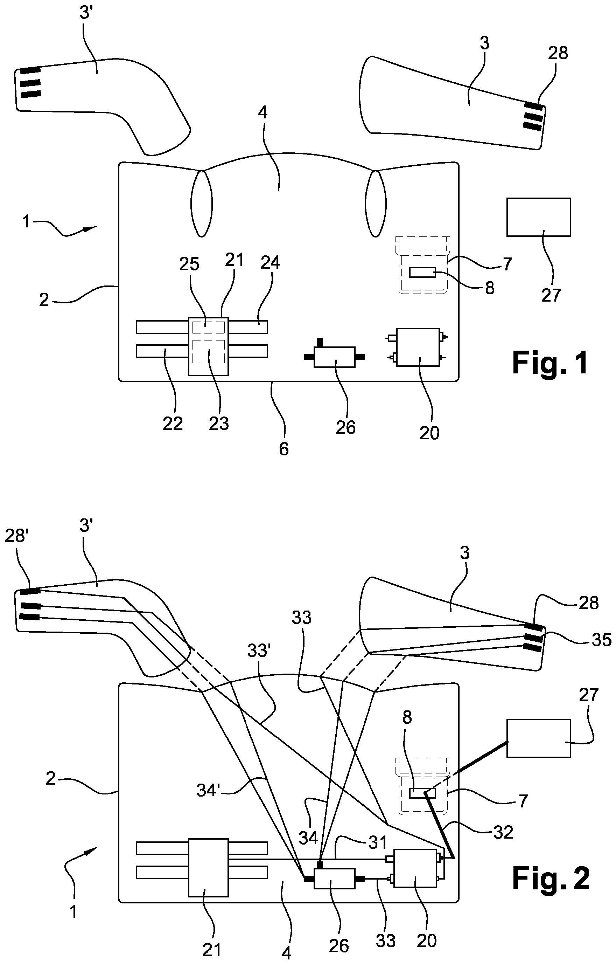

La veste 1, représentée sur la figure 1, comporte un corps principal 2, destiné à couvrir le buste d'un utilisateur, et des manches 3, 3'. Sur cette figure 1, la veste a été représentée en plan en position dépliée, avec les manches 3 détachées du corps 2 pour plus de clarté.

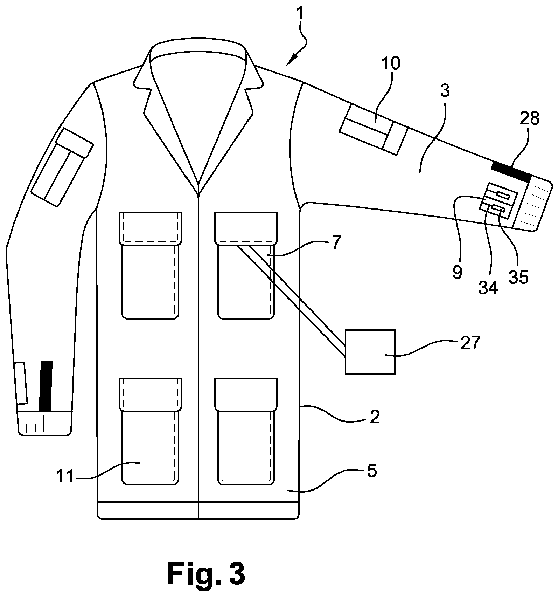

Le corps 2 de la veste se présente sous la forme d'une enveloppe externe constituée en matière textile, de préférence en une matière imperméable présentant une résistance mécanique importante et apte à évacuer la chaleur. Cette enveloppe comporte une face dite interne 4, destinée à être disposée en condition d'utilisation en regard du corps de l'utilisateur, qui est la face visible sur la figure 1, et une face externe opposée 5, visible sur la figure 3.

La veste 1 comporte en outre une doublure interne amovible, disposée en vis-à-vis de la face interne 4 de l'enveloppe externe. Cette doublure interne n'a pas été représentée sur les figures pour des raisons de clarté. Elle est constituée en matière textile, de préférence en une matière aérée permettant l'évacuation de la transpiration.

Les éléments constitutifs du système de contrôle non destructif permettant de réaliser les mesures de contrôle sont intégrés à la veste 1.

Contre la face interne 4 de l'enveloppe externe 2, entre cette dernière et la doublure interne, sont fixés une carte électronique de commande 20, ou carte mère, une source d'alimentation électrique 21 et un dispositif électronique de mesure 26.

Ces éléments sont avantageusement disposés de manière répartie sur la largeur de la veste, comme illustré sur la figure 1, de sorte à équilibrer le mieux possible le poids de la veste sur toute sa circonférence.

La carte électronique 20 est par exemple disposée dans une poche interne de l'enveloppe externe 2. De façon générale, elle est choisie de sorte à être légère et compacte, à présenter un faible besoin en alimentation électrique, et à permettre la connexion de multiples connecteurs. Cette carte électronique est dédiée à la commande de l'électronique de contrôle non destructif, ainsi qu'au traitement des résultats de l'inspection.

La carte électronique 20 est alimentée électriquement par la source d'alimentation électrique, ou batterie électrique portative 21. Cette dernière est classique en elle-même, et choisie conformément à l'invention pour présenter le meilleur compromis possible entre poids et autonomie de fonctionnement.

La veste 1 est pourvue d'un dispositif de réglage de la position de la batterie électrique 21, qui comporte de préférence des moyens de guidage du déplacement de la batterie entre des positions déterminées contre la surface de l'enveloppe externe 2, et des moyens d'immobilisation réversible dans chacune des ces positions.

A titre d'exemple non limitatif, ce dispositif comporte une glissière de guidage 22, fixée contre la face interne 4 de l'enveloppe externe 2, de préférence sensiblement parallèlement à un bord inférieur 6 de cette dernière, c'est-à-dire, dans une position normale d'utilisation de la veste, sensiblement horizontale. La batterie 21 est équipée, sur sa face en vis-à-vis de l'enveloppe externe 2, d'un crochet 23 en prise autour de la glissière 22, si bien qu'elle est apte à coulisser le long de cette dernière.

L'immobilisation réversible de la batterie 21 à différentes positions le long de la glissière 22 est assurée par des moyens d'accrochage coopérants fixés respectivement sur l'enveloppe externe 2 et sur la batterie 21. Ces moyens d'accrochage sont notamment du type auto-agrippants. Dans le mode de réalisation préféré représenté sur la figure 1, l'enveloppe externe 2 comporte, toujours sur sa face interne 4, une bande 25 du type à boucles ou crochets fixée sensiblement parallèlement à la glissière 22, et couvrant de préférence toute la longueur de cette dernière. La batterie 21 est munie, en sous-face, d'une bande 25 du type à crochets ou boucles complémentaires, permettant sa fixation, par simple pression exercée sur elle en direction de l'enveloppe externe 2, contre cette dernière, dans la position souhaitée.

La veste 1 comporte en outre un dispositif électronique de mesure 26, disposé par exemple dans une poche interne. Ce dispositif peut être de tout type connu de l'homme du métier, qui saura le choisir en fonction de l'application donnée, en association avec un ou une pluralité de capteurs de mesure adéquats. Il peut notamment s'agir d'un dispositif de mesure par ultrasons, ou encore par exemple par courant de Foucault. Il s'avère tout à fait avantageux dans le cadre de l'invention de choisir un dispositif électronique compact, léger et à basse consommation. Ce dispositif électronique 26 est connecté à la carte mère 20, qui assure d'une part son paramétrage et d'autre part la réception des données de mesure qui en sont issus. Un dispositif de mesure tel que celui décrit dans le document de brevet FR-A-2 874 689 est notamment particulièrement adapté pour une utilisation dans le cadre du système selon l'invention.

Le système comporte en outre un écran de commande et de visualisation 27, pour le paramétrage des mesures à effectuer par l'opérateur et l'affichage des résultats de ces mesures.

Cet écran 27 est disposé, par rapport à la veste 1, de sorte à pouvoir être aisément manipulé par l'opérateur et visible au moment des opérations de mesure.

Dans des variantes de l'invention, cet écran est mobile et relié à la carte électronique 20 par une liaison filaire de grande longueur, si bien qu'il peut être déplacé à distance de la veste 1 pour assurer la commodité de son utilisation. La veste 1 est pourvue, sur sa face externe 5, d'une poche 7 de réception de l'écran, par exemple au niveau de la poitrine à l'avant de la veste. L'écran 27 est apte à être rangé commodément dans la poche 7, dont il est extrait lorsqu'il est besoin de l'utiliser. Cette poche est de préférence doublée d'une couche de protection, par exemple en mousse.

Dans d'autres variantes de l'invention, qui ne sont pas incompatibles avec les précédentes, l'écran 27 est un écran flexible et/ou tactile qui est intégré dans l'enveloppe externe 2, par exemple sur une manche de la veste.

La veste 1 intègre également des moyens d'éclairage, par exemple sous forme de diodes électroluminescentes 28, qui sont disposées de sorte à éclairer à l'extérieur de la face externe 5 de la veste, de préférence au niveau d'une ou de chacune des deux manches 3, 3' de la veste, et à proximité du poignet. Ces diodes permettent avantageusement à l'opérateur d'éclairer la zone dans laquelle il effectue des mesures, près de sa main tenant le capteur de mesure, et ce sans générer pour lui de contrainte particulière. Les diodes 28 sont associées à des moyens de commande de leur allumage et de leur extinction, disposés à proximité d'elles, par exemple sur la manche 3, de sorte à être facilement accessibles par l'utilisateur portant la veste.

Les câbles de liaison électrique et électronique des différents éléments constitutifs ci-dessus sont représentés sur la figure 2. De façon générale, ces câbles sont constitués en matière textile, flexibles et plats. Ils peuvent le cas échéant être cousus contre la face interne 4 de l'enveloppe externe 2, afin de minimiser les risques de détérioration lors de la manipulation de la veste.

Un premier câble 31 relie la batterie 21 à la carte électronique 20.

Cette dernière est également reliée, d'une part à l'écran 27 par un câble 32, et d'autre part au dispositif électronique de mesure 26 par un câble 33.

L'enveloppe externe 2 est percée, à l'intérieur de la poche 7 de réception de l'écran 27, d'un orifice 8 pour le passage du câble 32 de l'intérieur vers l'extérieur de la veste.

La carte électronique 20 est en outre reliée, par des câbles 33, 33', aux diodes 28, 28' disposées respectivement au niveau des manches 3, 3' de la veste.

L'ensemble est complété par au moins un câble 34 destiné à connecter le dispositif électronique de mesure 26 à un capteur de mesure. Ce capteur de mesure, qui n'est pas représenté sur les figures, est classique en lui-même et choisi en fonction de l'application visée. Il est du type des capteurs à main, c'est-à-dire des capteurs qui sont tenus dans la main pour les approcher de la zone où doit être effectué le contrôle. A son extrémité de connexion au capteur, le câble 34 se termine par un connecteur 35 disposé sur la veste de sorte à être accessible, pour la connexion, depuis l'extérieur de la veste. Ce connecteur est disposé au niveau d'une manche 3, de préférence à proximité du poignet, c'est-à-dire au plus près de la main qui tiendra le capteur lors des opérations de mesure.

Dans des modes de réalisation préférés l'Invention prévoit en outre que le dispositif électronique de mesure 26 soit associé de la sorte à une pluralité de câbles de connexion à des capteurs de mesure, et de préférence à des câbles 34, 34' se terminant respectivement au niveau de chacune des manches 3, 3' de la veste, de sorte à permettre une utilisation aisée pour tout opérateur, qu'il soit droitier ou gaucher.

Le connecteur 35 a été représenté sur la figure 3, qui illustre un exemple de réalisation d'une veste 1 selon l'invention, refermée sur elle-même, en vue générale. Le connecteur apparaît à travers une fenêtre 9 réalisée dans l'enveloppe externe 2 au niveau de la manche 3, à l'extrémité du câble de liaison 34.

La veste 1 comporte en outre, sur sa face externe 5, de préférence au niveau de la manche 3, comme représenté sur la figure 3, une pochette 10 de réception d'un capteur de mesure à connecter au dispositif électronique de mesure 26 au moment de l'inspection.

La veste peut également comporter d'autres poches 11, pour recevoir tout autre élément utile à l'inspection, par exemple des capteurs supplémentaires.

Par un choix adéquat des éléments constitutifs, cette veste présente un poids limité, pouvant être aussi bas que 2 kg.

Lorsqu'il doit réaliser des opérations de contrôle non destructif, par exemple la mesure de l'épaisseur d'une pièce, l'opérateur enfile la veste 1. Son déplacement jusqu'à la zone où doit être réalisée l'inspection est facilité par le fait que le poids à supporter est réparti autour de son corps. Ses mains restent libres, si bien qu'il peut facilement se glisser dans des zones étroites ou périlleuses, dans des conditions de sécurité améliorées.

Les éléments électroniques constitutifs du système de contrôle sont en outre protégés entre l'enveloppe externe et la doublure interne de la veste.

Lorsqu'il s'apprête à effectuer les mesures d'inspection, l'opérateur extrait le capteur de sa pochette de réception 10 et le connecte au connecteur adéquat 35. Il extrait également l'écran de commande et de visualisation 27 de sa poche de réception 7 et, après l'avoir placé à l'endroit le plus adéquat pour son utilisation, il effectue le paramétrage des mesures. Il allume si besoin les diodes 28, et il dispose ensuite de ses deux mains pour tenir le capteur et assurer en même temps sa propre sécurité. Le câble 34 de liaison du capteur au dispositif électronique de mesure 26 étant entièrement inclus à l'intérieur de la veste 1, et n'en dépassant qu'au niveau du poignet, dans la fenêtre 9, il ne gêne avantageusement pas l'opérateur dans ses mouvements. Les mesures sont ainsi effectuées dans des conditions optimales, ce qui a pour effet d'augmenter leur fiabilité.

Une fois les mesures terminées, il ne reste plus à l'opérateur qu'à remettre le capteur et l'écran en place sur la veste et à regagner le poste dans lequel sera effectué le transfert des données de l'inspection de la carte électronique 20 vers un ordinateur prévu à cet effet.

La description ci-avant illustre clairement que par ses différentes caractéristiques et leurs avantages, la présente invention atteint les objectifs qu'elle s'était fixés. En particulier, elle fournit un système de contrôle non destructif qui assure, par rapport aux systèmes de l'art antérieur, un gain tant en temps qu'en sécurité et en confort pour l'utilisateur, et en fiabilité des mesures effectuées.

## Revendications

1. Système de contrôle non destructif, comportant en tant qu'éléments constitutifs un dispositif électronique de mesure non destructive (26) apte à être connecté à un capteur de mesure non destructive du type destiné à être tenu à la main pour la réalisation des mesures de contrôle, une carte électronique (20), une source d'alimentation électrique (21), un écran de visualisation et de commande (27), ledit dispositif électronique de mesure non destructive (26) étant relié à la carte électronique (20) elle-même reliée à la source d'alimentation électrique (21) et à l'écran de visualisation et de commande (27), et des câbles de liaison électrique et électronique desdits éléments constitutifs les uns aux autres, **caractérisé en ce que** ledit système de contrôle non destructif comporte un vêtement de type veste (1) et lesdits éléments constitutifs et lesdits câbles de liaison sont intégrés dans ledit vêtement de type veste (1), et **en ce qu'**un câble (34) de liaison du dispositif électronique de mesure (26) et d'un capteur de mesure comporte, à une extrémité de connexion audit capteur, un connecteur (35) disposé à l'extérieur de la veste (1), au niveau d'une manche (3) de ladite veste.

2. Système selon la revendication 1, **caractérisé en ce que** ledit connecteur (35) est disposé au niveau de ladite manche (3) à proximité du poignet.

3. Système selon la revendication 1 ou 2, **caractérisé en ce que** le dispositif électronique de mesure (26), la carte électronique (20), la source d'alimentation électrique (21) et les câbles de liaison sont disposés sur une face dite interne (4) de ladite veste (1) destinée à être positionnée en regard du corps d'un utilisateur.

4. Système selon la revendication 3, **caractérisé en ce que** ladite veste (1) comporte une enveloppe externe (2) et une doublure interne amovible, et **en ce que** le dispositif électronique de mesure (26), la carte électronique (20), la source d'alimentation électrique (21) et les câbles de liaison sont disposés entre ladite enveloppe externe et ladite doublure interne.

5. Système selon l'une quelconque des revendications 1 à 4, **caractérisé en ce que** la veste (1) comporte, sur une face dite externe (5), une poche (7) de réception d'un écran de visualisation et de commande (27), et **en ce que** ladite veste est percée, à l'intérieur de ladite poche, d'un orifice (8) pour le passage d'un câble (32) de liaison dudit écran et de ladite carte électronique (20).

6. Système selon l'une quelconque des revendications 1 à 5, **caractérisé en ce qu'**un écran de visualisation et de commande est intégré dans une manche (3) de ladite veste (1).

7. Système selon l'une quelconque des revendications 1 à 6, **caractérisé en ce que** la veste (1) comporte, sur une face dite externe (5), une pochette de réception dudit capteur de mesure.

8. Système selon l'une quelconque des revendications 1 à 7, **caractérisé en ce qu'**il comporte des moyens d'éclairage (28) disposés sur une face dite externe (5) de la veste (1).

9. Système selon la revendication 8, **caractérisé en ce que** lesdits moyens d'éclairage (28) sont disposés au niveau d'une manche (3) de ladite veste.

10. Système selon l'une quelconque des revendications 1 à 9, **caractérisé en ce qu'**il comporte un dispositif de réglage de la position de la source d'alimentation électrique (21) dans ladite veste (1).

11. Système selon la revendication 10, **caractérisé en ce que** ledit dispositif de réglage comporte des moyens (22, 23) de guidage du déplacement de ladite source d'alimentation électrique (21) entre des positions déterminées sur la veste (1), et des moyens (24, 25) d'immobilisation réversible dans chacune desdites positions déterminées.

## Patentansprüche

1. System zur zerstörungsfreien Prüfung, das als Bestandteile eine elektronische Vorrichtung zur zerstörungsfreien Messung (26), die mit einem Sensor zur zerstörungsfreien Messung der Art verbunden werden kann, die dazu bestimmt ist, zur Durchführung von Prüfmessungen in der Hand gehalten zu werden, eine Elektronikkarte (20), eine Stromversorgungsquelle (21), einen Anzeige- und Steuerbildschirm (27), wobei die elektronische Vorrichtung zur zerstörungsfreien Messung (26) mit der Elektronikkarte (20) verbunden ist, die selbst mit der Stromversorgungsquelle (21) und mit dem Anzeige- und Steuerbildschirm (27) verbunden ist, und Kabel zur elektrischen und elektronischen Verbindung der Bestandteile miteinander aufweist, **dadurch gekennzeichnet, dass** das System zur zerstörungsfreien Prüfung ein Kleidungsstück von der Art Jacke (1) aufweist, und die Bestandteile und die Verbindungskabel in das Kleidungsstück der Art Jacke (1) integriert sind, und dass ein Verbindungskabel (34) der elektronischen Messvorrichtung (26) und eines Messsensors an einem Anschlussende an den Sensor einen Verbinder (35) aufweist, der außerhalb der Jacke (1) im Bereich eines Ärmels (3) der Jacke angeordnet ist.

2. System nach Anspruch 1, **dadurch gekennzeichnet, dass** der Verbinder (35) im Bereich des Ärmels (3) in der Nähe des Handgelenks angeordnet ist.

3. System nach Anspruch 1 oder 2, **dadurch gekennzeichnet, dass** die elektronische Messvorrichtung (26), die Elektronikkarte (20), die Stromversorgungsquelle (21) und die Verbindungskabel auf einer so genannten Innenseite (4) der Jacke (1) angeordnet sind, die dazu bestimmt ist, gegenüber dem Körper eines Benutzers positioniert zu werden.

4. System nach Anspruch 3, **dadurch gekennzeichnet, dass** die Jacke (1) eine Außenhülle (2) und ein entfernbares Innenfutter aufweist, und dass die elektronische Messvorrichtung (26), die Elektronikkarte (20), die Stromversorgungsquelle (21) und die Verbindungskabel zwischen der Außenhülle und dem Innenfutter angeordnet sind.

5. System nach einem der Ansprüche 1 bis 4, **dadurch gekennzeichnet, dass** die Jacke (1) auf einer so genannten Außenseite (5) eine Tasche (7) zur Aufnahme eines Anzeige- und Steuerbildschirms (27) aufweist, und dass die Jacke im Inneren der Tasche eine Öffnung (8) für den Durchgang eines Verbindungskabels (32) des Bildschirms und der Elektronikkarte (20) hat.

6. System nach einem der Ansprüche 1 bis 5, **dadurch gekennzeichnet, dass** ein Anzeige- und Steuerbildschirm in einen Ärmel (3) der Jacke (1) integriert ist.

7. System nach einem der Ansprüche 1 bis 6, **dadurch gekennzeichnet, dass** die Jacke (1) auf einer so genannten Außenseite (5) ein Täschchen zur Aufnahme des Messsensors aufweist.

8. System nach einem der Ansprüche 1 bis 7, **dadurch gekennzeichnet, dass** es Beleuchtungseinrichtungen (28) aufweist, die auf einer so genannten Außenseite (5) der Jacke (1) angeordnet sind.

9. System nach Anspruch 8, **dadurch gekennzeichnet, dass** die Beleuchtungseinrichtungen (28) im Bereich eines Ärmels (3) der Jacke angeordnet sind.

10. System nach einem der Ansprüche 1 bis 9, **dadurch gekennzeichnet, dass** es eine Einstellvorrichtung der Position der Stromversorgungsquelle (21) in der Jacke (1) aufweist.

11. System nach Anspruch 10, **dadurch gekennzeichnet, dass** die Einstellvorrichtung Einrichtungen (22, 23) zur Führung der Verschiebung der Stromversorgungsquelle (21) zwischen bestimmten Positionen auf der Jacke (1) und Einrichtungen (24, 25) zur umkehrbaren Arretierung in jeder der bestimmten Positionen aufweist.

## Claims

1. Non-destructive testing system including by way of constituent elements an electronic non-destructive measuring device (26) able to be connected to a non-destructive measuring sensor of the type intended to be held in the hand in order to carry out test measurements, an electronic board (20), an electrical power source (21), and a control and display screen (27), said electronic non-destructive measuring device (26) being connected to the electronic board (20) which itself is connected to the electrical power source (21) and to the control and display screen (27), and cables for electrically and electronically linking said constituent elements to one another, **characterized in that** said non-destructive testing system includes a jacket-type garment (1) and said constituent elements and said linking cables are integrated into said jacket-type garment (1), and **in that** a cable (34) linking the electronic measuring device (26) and a measuring sensor includes, at an end for connecting to said sensor, a connector (35) placed on the exterior of the jacket (1), on a sleeve (3) of said jacket.

2. System according to Claim 1, **characterized in that** said connector (35) is placed on said sleeve (3) in proximity to the wrist.

3. System according to Claim 1 or 2, **characterized in that** the electronic measuring device (26), the electronic board (20), the electrical power source (21) and the linking cables are placed on what is called an internal face (4) of said jacket (1), intended to be positioned facing the body of a user.

4. System according to Claim 3, **characterized in that** said jacket (1) includes an external shell (2) and a removable internal lining, and **in that** the electronic measuring device (26), the electronic board (20), the electrical power source (21) and the linking cables are placed between said external shell and said internal lining.

5. System according to any one of Claims 1 to 4, **characterized in that** the jacket (1) includes, on what is called an external face (5), a pocket (7) for receiving a control and display screen (27), and **in that** said jacket is pierced, in the interior of said pocket, with an orifice (8) for the passage of a cable (32) linking said screen and said electronic board (20).

6. System according to any one of Claims 1 to 5, **characterized in that** a control and display screen is integrated into a sleeve (3) of said jacket (1).

7. System according to any one of Claims 1 to 6, **characterized in that** the jacket (1) includes, on what is called an external face (5), a small pocket for receiving said measuring sensor.

8. System according to any one of Claims 1 to 7, **characterized in that** it includes lighting means (28) placed on what is called an external face (5) of the jacket (1).

9. System according to Claim 8, **characterized in that** said lighting means (28) are placed on a sleeve (3) of said jacket.

10. System according to any one of Claims 1 to 9, **characterized in that** it includes a device for adjusting the position of the electrical power source (21) in said jacket (1).

11. System according to Claim 10, **characterized in that** said adjusting device includes means (22, 23) for guiding the movement of said electrical power source (21) between defined positions in the jacket (1), and means (24, 25) for reversibly immobilizing the source in each of said defined positions.
